(19) Europäisches Patentamt / European Patent Office / Office européen des brevets

(11) **EP 4 327 739 B1**

(12) **EUROPEAN PATENT SPECIFICATION**

(45) Date of publication and mention
of the grant of the patent:
**29.10.2025 Bulletin 2025/44**

(21) Application number: **23188065.9**

(22) Date of filing: **27.07.2023**

(51) International Patent Classification (IPC):
***A61B 5/11*** *(2006.01)*

(52) Cooperative Patent Classification (CPC):
**A61B 5/1123; A61B 5/681; A61B 5/7207;**
A61B 2562/0219

(54) **ELECTRONIC DEVICE, METHOD, AND PROGRAM FOR DETERMINING AN ARM SWING DIRECTION**

ELEKTRONISCHE VORRICHTUNG, VERFAHREN UND PROGRAMM ZUR BESTIMMUNG EINER BEWEGUNGSRICHTUNG EINES ARMES

DISPOSITIF ÉLECTRONIQUE, PROCÉDÉ ET PROGRAMME POUR LA DÉTERMINATION D'UNE DIRECTION DE MOUVEMENT D'UN BRAS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **26.08.2022 JP 2022134890**

(43) Date of publication of application:
**28.02.2024 Bulletin 2024/09**

(73) Proprietor: **Casio Computer Co., Ltd.**
**Tokyo 151-8543 (JP)**

(72) Inventor: **Ide, Hiroyasu**
**Hamura-shi, Tokyo, 205-8555 (JP)**

(74) Representative: **Grünecker Patent- und Rechtsanwälte**
**PartG mbB**
**Leopoldstraße 4**
**80802 München (DE)**

(56) References cited:
**US-A1- 2021 005 071     US-B1- 10 049 595**
**US-B2- 10 371 516       US-B2- 10 921 342**

## Description

The present disclosure relates generally to an electronic device, a method, and a computer-readable storage medium for determining an arm swing direction.

In recent years, electronic devices that are worn on the arm and that detect the movement of the body of a user have been developed. Examples of such electronic devices include wearable terminals such as smartwatches, activity trackers, and the like. One technology used in such electronic devices involves calculating forward-backward acceleration of the user from acceleration data obtained by an acceleration sensor, and counting a number of steps by counting, as one step, points in time at which the calculated forward-backward acceleration is zero.

Document US 10,371,516 B2, which discloses the subject matter of the preambles of the independent claims, describes a method and apparatus for determining the misalignment between a device held by a pedestrian and the pedestrian. The device includes a sensor assembly including, in particular, accelerometers and/or gyroscopes. The misalignment is meant as a misalignment between a frame of the sensor assembly and a frame of the pedestrian. The present invention is defined by the subject matter of the appended independent claims.

A more complete understanding of this application can be obtained when the following detailed description is considered in conjunction with the following drawings, in which:

FIG. 1 is a block diagram illustrating an example of the functional configuration of an electronic device according to an embodiment;
FIG. 2 is a drawing for explaining various axes of acceleration detected by a detector in a case in which the electronic device according to the embodiment is worn on a left wrist of a user;
FIG. 3 is a drawing illustrating an example of the acceleration detected by the electronic device according to the embodiment;
FIG. 4 is an example of a flowchart of arm swing determination processing according to the embodiment;
FIG. 5 is a drawing for explaining x2 axis acceleration calculated in the arm swing determination processing according to the embodiment;
FIG. 6 is a drawing for explaining a horizontal acceleration ha and the like calculated in the arm swing determination processing according to the embodiment;
FIG. 7 is a drawing illustrating an example of a norm n and a leveling horizontal signal Lha calculated in the arm swing determination processing according to the embodiment;
FIG. 8 is a drawing illustrating an example of horizontal velocity obtained in the arm swing determination processing according to the embodiment;
FIG. 9 is a drawing illustrating an example of the acceleration detected by the electronic device according to the embodiment in a case in which the user walks while bending the arm at substantially a right angle;
FIG. 10 is a drawing illustrating an example of the norm n and the leveling horizontal signal Lha calculated in the arm swing determination processing according to the embodiment in a case in which the user walks while bending the arm at substantially a right angle; and
FIG. 11 is a drawing illustrating an example of horizontal velocity obtained in the arm swing determination processing according to the embodiment in a case in which the user walks while bending the arm at substantially a right angle.

Hereinafter, an electronic device and the like according to various embodiments are described while referencing the drawings. Note that, in the drawings, identical or corresponding components are denoted with the same reference numerals.

Embodiments

An electronic device according to this embodiment is an information processing device that can be worn on an arm of a user and that can determine a form of an exercise by an acceleration sensor. In one example, the electronic device according to this embodiment is a smartwatch or an electronic watch.

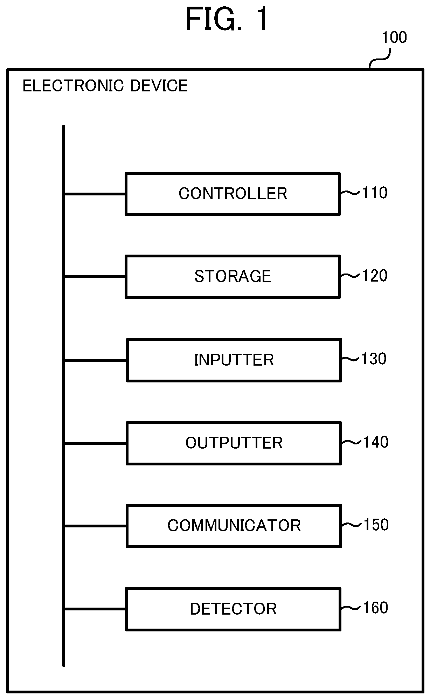

As illustrated in FIG. 1, an electronic device 100 includes a controller 110, a storage 120, an inputter 130, an outputter 140, a communicator 150, and a detector 160.

In one example, the controller 110 is configured from a processor such as a central processing unit (CPU) or the like. The controller 110 executes, by a program stored in the storage 120, processing for realizing the various functions of the electronic device 100, hereinafter described arm swing determination processing, and the like. The controller 110 is compatible with multithreading, and can execute a plurality of processes in parallel.

The storage 120 stores programs to be executed by the controller 110 and necessary data. The storage 120 can include random access memory (RAM), read-only memory (ROM), flash memory, and the like, but the storage 20 is not limited thereto. Note that the storage 120 may be provided inside the controller 110.

The inputter 130 is a user interface such as a push button switch, a touch panel, or the like, and receives operations/inputs from the user. When the inputter 130 includes a touch panel, the touch panel may be integrated with a display of the outputter 140.

The outputter 140 includes a display such as a liquid crystal display, an organic electroluminescence (EL) display, or the like, and displays display screens, operation screens, and the like that provide the functions of the electronic device 100.

In one example, the communicator 150 is implemented as network interface that is compatible with a wireless local area network (LAN), long term evolution (LTE), or the like. The electronic device 100 can communicate with the internet and other devices via the communicator 150.

The detector 160 detects exercise information of the user. In the present invention, acceleration information is used as the exercise information of the user. Accordingly, the detector 160 includes an acceleration sensor that detects acceleration in each direction of three axes (X-axis, Y-axis, Z-axis) that are orthogonal to each other. It is preferable that, in the acceleration sensor, the acceleration is detected using a sampling frequency of about 25 Hz to 100 Hz. The controller 110 can acquire, as a detected value and at a desired timing (note that the temporal resolution depends on the sampling frequency), a value detected by the acceleration sensor of the detector 160.

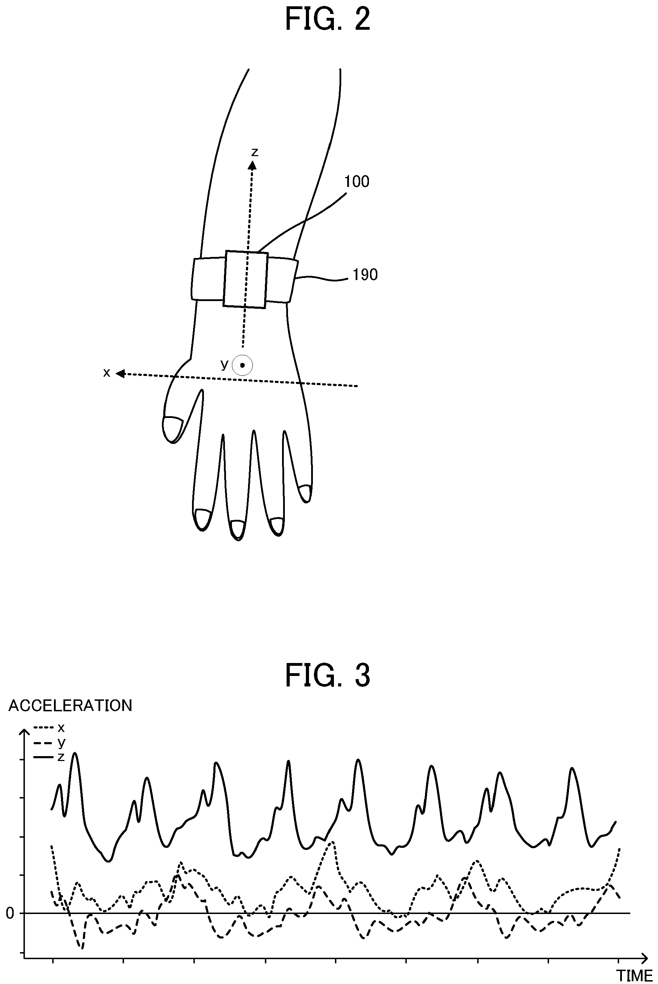

As illustrated in FIG. 2, the electronic device 100 is worn on a wrist of the user using a band 190. The arm on which the electronic device 100 is worn may be the right arm or the left arm but, in this case, as illustrated in FIG. 2, a case is described in which the electronic device 100 is worn on the back side of the left hand. Note that whether the electronic device 100 is worn on the left or right arm may be input via the inputter 130 or the like and stored in the storage 120.

In this case, the various axes of the acceleration sensor of the detector 160 are, as illustrated in FIG. 2, an x axis, which is a direction from the little finger toward the thumb, a y axis, which is a direction from the side of the wrist with the palmaris longus muscle (palm side) toward the back side thereof (the back side of the hand), and a z axis, which is a direction from the hand toward the elbow. Note that, although gravity is a downward force, gravitational acceleration is indistinguishable from acceleration that occurs when an object is accelerating upwards at 1G in zero gravity space. As such, the acceleration sensor detects the gravitational acceleration as vertically upward acceleration.

When, as illustrated in FIG. 2, the user walks while wearing the electronic device 100, the acceleration on each axis is acquired by the detector 160 as a discrete signal sampled at the sampling frequency. When the user walks without bending the elbow, the acceleration on each axis is acquired as illustrated in FIG. 3, for example. In this case, frequently, the z axis value that is affected by the gravitational acceleration is the greatest, and the x axis value that is affected by the forward-backward swinging of the hand is the next greatest. Additionally, since the hand rarely swings from left to right when walking, frequently, the y axis value is comparatively less.

Accordingly, in such a case, it is not strange to think that an arm swing direction can be determined on the basis of the x axis acceleration value. However, in reality, due to shifting of a wearing position of the electronic device 100, the manner in which the user swings their arm, and the like, the x axis may not reflect only the forward-backward swinging of the arm. As such, when determining the arm swing direction, the electronic device 100 splits the values of the acceleration on the three axes detected by the detector 160 into a vertical direction (gravitational direction) and a direction orthogonal to the vertical direction (horizontal direction), acquires a horizontal velocity from the latter value (horizontal acceleration), and determines the arm swing direction on the basis of this horizontal velocity.

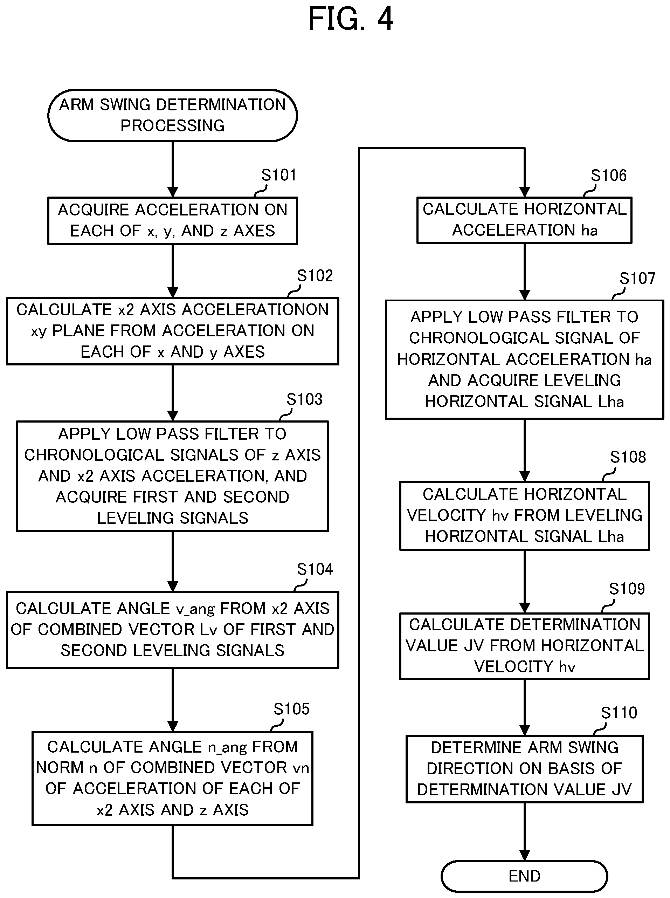

Arm swing determination processing for determining the arm swing direction on the basis of the values of the acceleration on the three axes is described while referencing FIG. 4. This processing may be executed in parallel with other necessary processings when the electronic device 100 is started up, or may be executed upon receipt of a command input by the user via the inputter 130 to perform the arm swing determination.

Firstly, the controller 110 acquires, from the detector 160, an acceleration value of each of the x axis, the y axis, and the z axis (x axis signal, y axis signal, and z axis signal) (step S101). Next, the controller 110 calculates, from the x axis component (x axis signal) and the y axis component (y axis signal) of the acceleration, an x2 axis acceleration (x2 signal) on the xy plane (step S102).

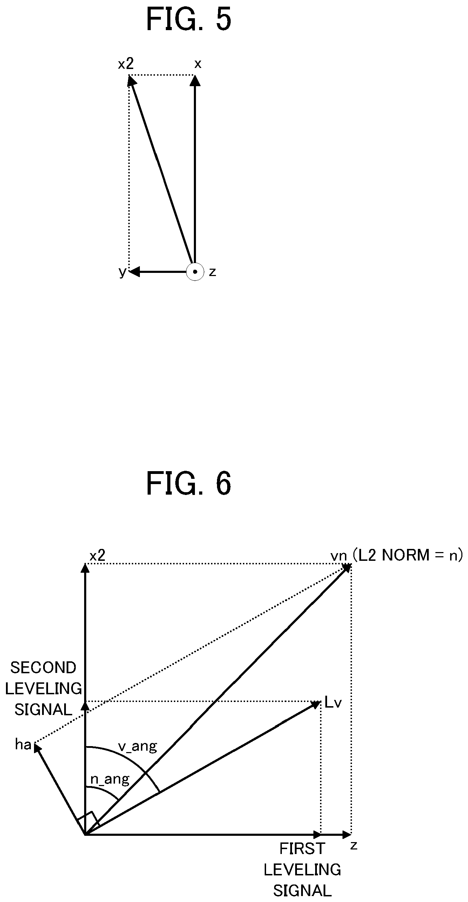

As illustrated in FIG. 5, the x2 axis acceleration is a sum vector of the x axis direction vector and the y axis direction vector of the acceleration, and this sum vector is orthogonal to the z axis. Moreover, a value (scalar value) of the x2 axis acceleration (x2 signal) is an L2 norm of a vector obtained by combining the x axis direction and the y axis direction of the acceleration. For the sake of convenience, the reference symbol of the x axis acceleration is applied as the reference symbol of the x2 axis acceleration (x2 signal). That is, when the x axis signal at a time i is expressed as xi and the y axis signal at the time i is expressed as yi, the x2 signal (x2i) is calculated by Equation (1) below. Note that sign(x) is a function that returns 1 when the reference symbol of x is positive and -1 when the reference symbol of x is negative.

$$x2i = \sqrt{(xi^2 + yi^2)} \times \text{sign}(xi) \quad (1)$$

Next, the controller 110 applies a low pass filter (LPF) that cuts high-frequency components greater than or equal to a first reference frequency (0.05 Hz to 1 Hz, for example, 0.1 Hz) to each of a signal (first signal) expressing chronological data of the z axis acceleration (first acceleration component that is an acceleration component of a first axis (z axis) selected from the three axes), and a signal (second signal) expressing chronological data of the x2 axis acceleration (second acceleration component that is a component of the sum of the two axes (the x axis and the y axis) other than the first axis), and acquires a first leveling signal (LPF-applied zi) and a second leveling signal (LPF-applied x2i) (step S103).

Then, the controller 110 calculates an angle v_ang from the x2 axis of a leveling vector Lv that is a combined vector of a vector expressed by the first leveling signal and a vector expressed by the second leveling signal (step S104). That is, when the x2 signal at the time i is expressed as x2i and the z axis signal at the time i is expressed as zi, v_angi is calculated by Equation (2) below.

$$\text{v\_angi} = \tan^{-1} \text{(LPF-applied zi/LPF-applied x2i)} \quad (2)$$

Next, the controller 110 calculates n, which is the L2 norm of the combined vector vn of the x axis signal (first acceleration component) and the x2 axis signal (second acceleration component), and n_ang, which is an angle from the x2 axis of vn (step S105). That is, when the x2 signal at the time i is expressed as x2i and the z axis signal at the time i is expressed as zi, ni and n_angi are calculated by Equations (2) and (3) below.

$$\text{ni} = \sqrt{(\text{x2i}^2 + \text{zi}^2)} \quad (3)$$

$$\text{n\_angi} = \tan^{-1}(\text{zi/x2i}) \quad (4)$$

Next, the controller 110 calculates a horizontal acceleration ha by taking the component of the direction in which the combined vector vn of the z axis signal (first acceleration component) and the x2 axis signal (second acceleration component) is orthogonal to the v_ang on the x2-z plane (step S106). That is, when the norm n at the time i is expressed as ni, the v_ang at the time i is expressed as v_angi, and the n_ang at the time i is expressed as n_angi, hai is calculated by Equation (5) below.

$$\text{hai} = \text{ni} \times \sin(\text{n\_angi} - \text{v\_angi}) \times (-1) \quad (5)$$

The relationships between the various values described above are illustrated in FIG. 6. By applying the low pass filter to the x axis acceleration (first acceleration component) and the x2 axis acceleration (second acceleration component), various types of noise components caused by walking and arm swinging are removed and the gravitational acceleration becomes the dominant value. As such, the leveling vector Lv, which is the combined vector of the vector expressed by the first leveling signal and the vector expressed by the second leveling signal, becomes a vertical (average gravitational direction) vector. Accordingly, a vector ha, which is a vector perpendicular to the leveling vector Lv can be thought of as a horizontal vector, and it is understood that the horizontal acceleration is expressed by the vector ha.

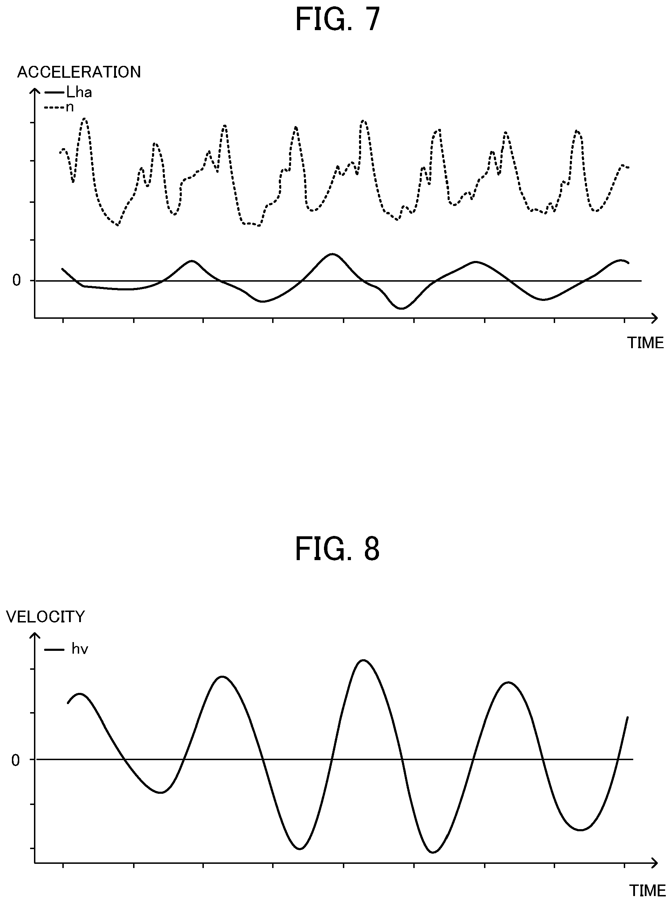

Next, in order to remove the high-frequency noise components of the horizontal acceleration ha, the controller 110 applies a low pass filter that cuts high-frequency components greater than or equal to a second reference frequency (1.5 Hz to 3 Hz, for example, 2 Hz) to a signal expressing chronological data of the horizontal acceleration ha, and acquires a leveling horizontal signal Lha (step S107). For example, when the norm n of the combined vector vn and the leveling horizontal signal Lha are calculated from the signal (chronological signal) expressing the chronological data of the acceleration on the three axes illustrated in FIG. 3, a graph such as illustrated in FIG. 7 is obtained.

Next, the controller 110 integrates the leveling horizontal signal Lha to calculate a horizontal velocity hv (step S108). Note that the leveling horizontal signal Lha also includes, as noise components, signals other than horizontal signals, and signals caused by walking or running (caused by movement of the user). As such, in order to suppress the influence of these noise components, the controller 110 may calculate a horizontal acceleration moving average (MAha) that is a moving average of the leveling horizontal signal Lha, and may calculate the horizontal velocity hv by subtracting the horizontal acceleration moving average MAha from the leveling horizontal signal Lha and then integrating.

Then, the controller 110 calculates a determination value JV for determining the arm swing direction from the horizontal velocity hv (step S109). Here, the controller 110 may use the horizontal velocity hv as the determination value JV without modification. However, the horizontal velocity hv also includes, as noise components, components other than the horizontal components, and components caused by walking or running (caused by movement of the user). Accordingly, in order to suppress the influence of these noise components, the controller 110 may calculate a horizontal velocity moving average MAhv that is a moving average of the horizontal velocity hv, and may calculate the determination value JV by subtracting the horizontal velocity moving average MAhv from the horizontal velocity hv.

Next, the controller 110 determines the arm swing direction on the basis of the determination value JV (step S110), and ends the arm swing determination processing. A value of the determination value JV (that is, the horizontal velocity hv) takes a positive value when the arm is moving in the forward direction, and takes a negative value when the arm is moving in the backward direction. Moreover, the value is 0 at the point in time of a turn-back of the arm swing. For example, when calculating the horizontal velocity hv from the leveling horizontal signal Lha illustrated in FIG. 7, a graph such as illustrated in FIG. 8 is obtained. In this graph, the arm swing direction switches at the timing at which the value is 0, the arm swing direction is the forward direction while the value is positive, and the arm swing direction is the backward direction while the arm swing direction is negative. Accordingly, the controller 110 can determine the arm swing direction on the basis of the value of the horizontal velocity hv (that is, the determination value JV).

Furthermore, the controller 110 can determine a ground contact foot from a ground contact timing of the foot and the determination value JV. For example, the controller 110 determines that a foot on the same side as the arm on which the electronic device 100 is worn is contacting the ground when, in a period from a ground contact timing of one foot to a ground contact timing of the other foot, an amount of time that the determination value JV takes a positive value is longer than an amount of time that the determination value JV takes a negative value.

Additionally, provided that whether the arm on which the electronic device 100 is worn is the left or the right is stored in advance in the storage 120 by inputting via the inputter 130 or the like, the ground contact foot can be determined (estimated) on the basis of at least the value that the determination value JV takes. For example, when the arm on which the electronic device 100 is worn is the left arm and the user is walking, a determination (estimation) is made that the left foot is the ground contact foot when the determination value JV switches from 0 to positive. Additionally, there is a high possibility that both feet are contacting the ground at the moment at which the determination value JV switches from 0 to positive, but the right foot ceases to be a ground contact foot when the determination value JV increases and can be determined (estimated) to be a ground contact foot again when the determination value JV decreases and the determination value JV becomes 0 again. Moreover, a determination (estimation) may be made that the arm is at the side of the body of the user when the determination value JV takes an extreme value. Regarding the aforementioned, a similar determination (estimation) can be performed when the determination value JV takes a negative value or when the wearing arm is the right arm.

Note that any method can be used to detect the ground contact of the foot. For example, the controller 110 can detect the ground contact of the foot by detecting a peak in a combined signal of the accelerations of all three axes detected by the detector 160. In such a case, the controller 110 also functions as a ground contact detector.

Due to the arm swing determination processing described above, the controller 110 can determine the arm swing direction of the user on the basis of the data of the acceleration on the 3 axes. Additionally, the controller 110 can determine the ground contact foot by detecting the ground contact timing of the foot.

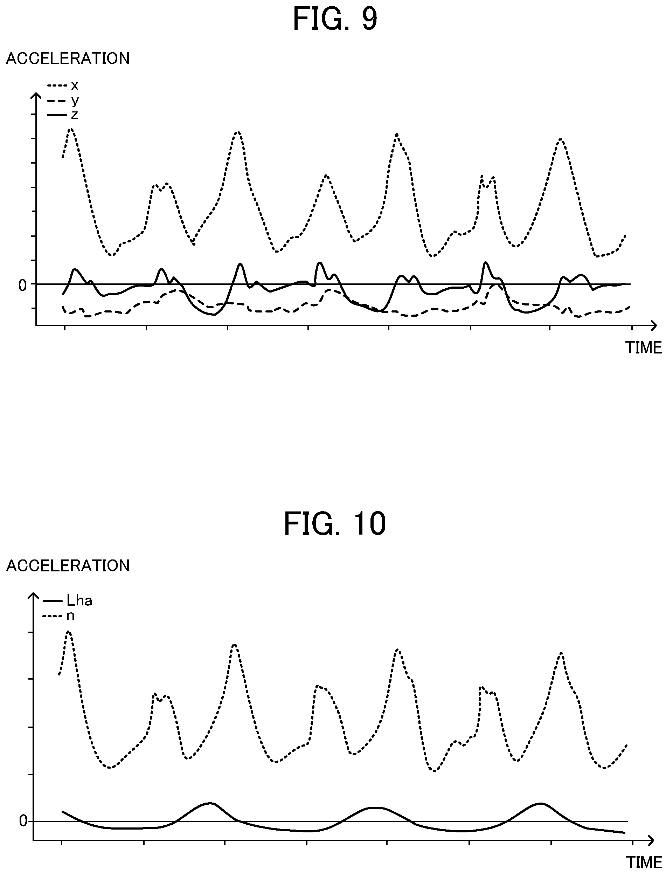

In the description given above, a case is described in which the user walks without bending the elbow. Next, a case is considered in which the user bends the elbow (for example, bends the arm at substantially a right angle) and walks. In this case, the x axis is most affected by the gravitational acceleration, the z axis is affected by the forward-backward swing of the arm, and acceleration on the various axes is acquired as illustrated in FIG. 9, for example.

Moreover, as understood from FIGS. 5 and 6, the magnitude of the norm n is greatly affected by the axis with the greatest amplitude among the three axes, the x axis, the y axis, and the z axis. Accordingly, a graph of the norm n along a time series is, as illustrated in FIG. 10, a graph that is similar to the x axis acceleration of FIG. 9.

Additionally, the horizontal acceleration ha is a vector perpendicular to the leveling vector Lv but, in this case as well, the various types of noise components are removed from the leveling vector Lv, and the gravitational acceleration becomes the dominant value. As such, the leveling vector Lv becomes a vertical (average gravitational direction) vector. Accordingly, even when the user bends the elbow and walks, a value of a vector perpendicular to the vertical direction (Lv) is obtained as the horizontal acceleration ha, and the controller 110 can acquire the leveling horizontal signal Lha such as illustrated in FIG. 10, for example, by leveling that value.

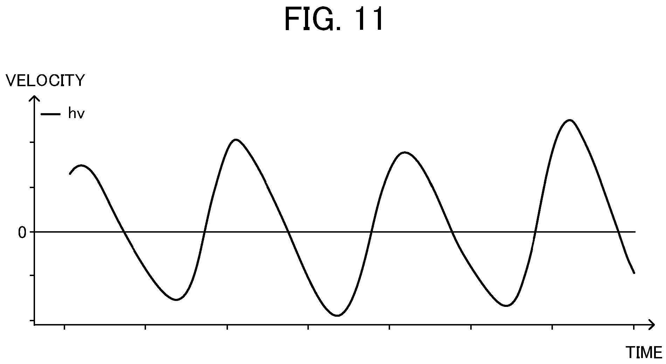

Moreover, the controller 110 can calculate the horizontal velocity hv such as illustrated in FIG. 11, for example, by integrating the leveling horizontal signal Lha.

Thus, even when the first axis is not the axis most affected by gravity (the z axis when the elbow is not bent, and the x axis with the elbow is bent), a substantially vertical vector can be obtained by calculating the leveling vector Lv, and the horizontal acceleration ha can be obtained by calculating a vector perpendicular to the leveling vector Lv.

Note that the technology described in Unexamined Japanese Patent Application Publication No. 2011-90548, which is a prior art document, calculates the forward-backward acceleration of the user from acceleration data obtained by an acceleration sensor, and counts the number of steps by counting, as one step, points in time at which the calculated forward-backward acceleration is zero. In reality, accurately calculating the forward-backward acceleration is difficult, but it is sufficient to detect the inversion of the reference numeral of the forward-backward acceleration to count the number of steps and, as such, it is possible to count the number of steps by this method without any problems. However, although the number of steps can be counted, this sort of method is unsuitable for determining (estimating) the actual form of the exercise. The present disclosure is made with the view of this situation, and enables the determination of the form of the exercise of the user on the basis of exercise information of the user.

Modified Examples

Note in the embodiment described above, a determination of the arm swing direction and a determination of the ground contact foot were performed as the determination of the form of the exercise of the user. However, a configuration is possible in which the controller 110 performs a form analysis (displaying the form of the exercise as an animation, offering improvement advice, or the like) of the exercise of the user on the basis of information about the determined arm swing and/or ground contact foot. Moreover, a configuration is possible in which the controller 110 sends the determined arm swing and/or ground contact foot, form analysis results, and the like to another device (smartwatch, smartphone, or the like) via the communicator 150, and displays the determined arm swing and/or contact foot, form analysis results, and the like on a display of the other device.

The electronic device 100 is not limited to a smartwatch, and can be realized by a smartphone provided with the detector 160, or a computer such as a portable tablet, a personal computer (PC), or the like. Specifically, in the embodiment described above, an example is described in which the program of the arm swing determination processing and the like executed by the controller 110 are stored in advance in the storage 120. However, a computer may be configured that is capable of executing the various processings described above by storing and distributing the programs on a non-transitory computer-readable recording medium such as a compact disc read-only memory (CD-ROM), a digital versatile disc (DVD), a magneto-optical, disc (MO), a memory card, and a USB memory, and reading out and installing these programs on the computer.

Furthermore, the program can be superimposed on a carrier wave and applied via a communication medium such as the internet. For example, the program may be posted to and distributed via a bulletin board system (BBS) on a communication network. Moreover, a configuration is possible in which the various processings described the above are executed by starting the programs and, under the control of the operating system (OS), executing the programs in the same manner as other applications/programs.

Additionally, a configuration is possible in which the controller 110 is constituted by a desired processor unit such as a single processor, a multiprocessor, a multi-core processor, or the like, or by combining these desired processors with processing circuity such as an application specific integrated circuit (ASIC), a field-programmable gate array (FPGA), or the like.

The foregoing describes some example embodiments for explanatory purposes. Although the foregoing discussion has presented specific embodiments, persons skilled in the art will recognize that changes may be made in form and detail within the scope of the invention as defined by the appended claims. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

**Claims**

1. An electronic device (100) configured to be worn on an arm of a user, the electronic device (100) comprising:

     a detector (160) configured to detect acceleration components of the user in each direction of three axes that are orthogonal to each other and a controller (110),
     **characterized in that**
     the controller (110) is configured to
     determine, based on the acceleration components detected by the detector (160), an arm swing direction of the user, wherein
     the controller (110) is configured to
     acquire, based on the acceleration components on the three axes detected by the detector (160), a horizontal velocity that is a horizontal velocity component,
     with the acquired horizontal velocity as a judgment value determines, based on the judgment value, the arm swing direction of the user,
     split the acceleration components on the three axes into a first acceleration component that is an acceleration component on a first axis selected from the three axes, and a second acceleration component that is a component of a sum of the acceleration components of two axes other than the first axis, and
     acquire, based on the first acceleration component and the second acceleration component, the horizontal velocity.

2. The electronic device (100) according to claim 1, wherein the controller (110)

calculates, based on a first leveling signal obtained by cutting high-frequency components greater than or equal to a first reference frequency in a first signal that is a signal expressing chronological data of the first acceleration component and a second leveling signal obtained by cutting the high-frequency components greater than or equal to the first reference frequency in a second signal that is a signal expressing chronological data of the second acceleration component, a horizontal acceleration, and
acquires the horizontal velocity from the calculated horizontal acceleration.

3. The electronic device (100) according to claim 2, wherein the controller (110) acquires the horizontal velocity from a leveling horizontal signal obtained by cutting high-frequency components greater than or equal to a second reference frequency in a signal expressing chronological data of the horizontal acceleration.

4. The electronic device (100) according to claim 3, wherein the controller (110)

calculates a horizontal acceleration moving average that is a moving average of the leveling horizontal signal, and
acquires the horizontal velocity by integrating a value obtained by subtracting the horizontal acceleration moving average from the leveling horizontal signal.

5. The electronic device (100) according to claim 4, wherein the controller (110)

calculates a horizontal velocity moving average that is a moving average of the horizontal velocity, and
sets a value obtained by subtracting the horizontal velocity moving average from the horizontal velocity as the judgment value.

6. The electronic device (100) according to claim 1, wherein the controller (110) determines, based on at least the determined arm swing direction, a ground contact foot of the user.

7. The electronic device (100) according to claim 6, wherein
the controller (110) detects a timing of a ground contact of a foot of the user, and determines, based on the detected timing of the ground contact and the determined arm swing direction, the ground contact foot of the user.

8. The electronic device (100) according to claim 7, wherein the controller (110) determines that a foot on a same side as the wearing arm is making ground contact when, in a period from the detected timing of the ground contact to a timing of a next ground contact, an amount of time in which the judgment value takes a positive value is greater than an amount of time in which the judgment value takes a negative value.

9. An arm swing direction determination method in which an information processing device is worn on an arm of a user and includes a detector (160) that detects acceleration components of the user in each direction of three axes that are orthogonal to each other, and a controller (110), the method being **characterized by** comprising:

determining, by the controller (110) and based on the acceleration components detected by the detector (160), an arm swing dirction of the user, wherein
the determining incudes
acquiring, based on the acceleration components on the three axes detected by the detector (160), a horizontal velocity that is a horizontal velocity component,
with the acquired horizontal velocity as a judgment value determining, based on the judgment value, the arm swing direction of the user,
splitting the acceleration components on the three axes into a first acceleration component that is an acceleration component on a first axis selected from the three axes, and a second acceleration component that is a component of a sum of the acceleration components of two axes other than the first axis, and
acquiring, based on the first acceleration component and the second acceleration component, the horizontal velocity.

10. A computer-readable storage medium containing a program **characterized by** causing a controller (110), of an information processing device that is configured to be worn on an arm of a user and that includes a detector (160) configured to detect acceleration components of the user in each direction of three axes that are orthogonal to each other and the controller (110), to execute processing for:

determining, based on the acceleration components detected by the detector (160), an arm swing direction of the user, wherein
the determining incudes
acquiring, based on the acceleration components on the three axes detected by the detector (160), a horizontal velocity that is a horizontal velocity component,
with the acquired horizontal velocity as a judgment value determining, based on the judgment value, the arm swing direction of the user,
splitting the acceleration components on the three axes into a first acceleration component that is an acceleration component on a first axis selected from the three axes, and a second acceleration component that is a component of a sum of the acceleration components of two axes other than the first axis, and
acquiring, based on the first acceleration component and the second acceleration component, the horizontal velocity.

**Patentansprüche**

1. Elektronische Vorrichtung (100), die dazu konfiguriert ist, an einem Arm eines Benutzers getragen zu werden, wobei die elektronische Vorrichtung (100) Folgendes umfasst:

   einen Detektor (160), der dazu konfiguriert ist, Beschleunigungskomponenten des Benutzers in jeder Richtung von drei zueinander orthogonalen Achsen zu ermitteln; und
   eine Steuerung (110),
   **dadurch gekennzeichnet, dass**
   die Steuerung (110) zu Folgendem konfiguriert ist:
   Bestimmen, basierend auf den von dem Detektor (160) ermittelten Beschleunigungskomponenten eine Armschwungrichtung des Benutzers, wobei die Steuerung (110) zu Folgendem konfiguriert ist:

      Erfassen, basierend auf den von dem Detektor (160) ermittelten Beschleunigungskomponenten auf den drei Achsen, einer horizontalen Geschwindigkeit, die eine horizontale Geschwindigkeitskomponente ist,
      mit der erfassten horizontalen Geschwindigkeit als Beurteilungswert, Bestimmen, basierend auf dem Beurteilungswert, der Armschwungrichtung des Benutzers,
      Aufteilen der Beschleunigungskomponenten auf den drei Achsen in eine erste Beschleunigungskomponente, die eine Beschleunigungskomponente auf einer ersten, aus den drei Achsen ausgewählten Achse ist, und eine zweite Beschleunigungskomponente, die eine Komponente einer Summe der Beschleunigungskomponenten von zwei anderen Achsen als der ersten Achse ist, und
      Erfassen, basierend auf der ersten Beschleunigungskomponente und der zweiten Beschleunigungskomponente, der horizontalen Geschwindigkeit.

2. Elektronische Vorrichtung (100) nach Anspruch 1, wobei die Steuerung (110)

   basierend auf einem ersten Nivellierungssignal, das durch Abschneiden von Hochfrequenzkomponenten, die größer oder gleich einer ersten Referenzfrequenz sind, in einem ersten Signal, das ein Signal ist, das chronologische Daten der ersten Beschleunigungskomponente ausdrückt, erhalten wird, und einem zweiten Nivellierungssignal, das durch Abschneiden der Hochfrequenzkomponenten, die größer oder gleich der ersten Referenzfrequenz sind, in einem zweiten Signal, das ein Signal ist, das chronologische Daten der zweiten Beschleunigungskomponente ausdrückt, erhalten wird, eine horizontale Beschleunigung berechnet, und
   die horizontale Geschwindigkeit aus der berechneten horizontalen Beschleunigung ermittelt.

3. Elektronische Vorrichtung (100) nach Anspruch 2, wobei die Steuerung (110) die horizontale Geschwindigkeit aus einem horizontalen Nivellierungssignal erfasst, das durch Abschneiden von Hochfrequenzkomponenten, die größer oder gleich einer zweiten Referenzfrequenz sind, in einem Signal erhalten wird, das chronologische Daten der horizontalen Beschleunigung ausdrückt.

4. Elektronische Vorrichtung (100) nach Anspruch 3, wobei die Steuerung (110)

   einen gleitenden Durchschnitt der horizontalen Beschleunigung berechnet, der ein gleitender Durchschnitt des horizontalen Nivellierungssignals ist, und
   die horizontale Geschwindigkeit durch Integration eines Wertes, der durch Subtraktion des gleitenden Durchschnitts der horizontalen Beschleunigung vom horizontalen Nivellierungssignal erhalten wird, erfasst.

5. Elektronische Vorrichtung (100) nach Anspruch 4, wobei die Steuerung (110)

einen gleitenden Durchschnitt der horizontalen Geschwindigkeit berechnet, der ein gleitender Durchschnitt der horizontalen Geschwindigkeit ist, und
einen Wert, der durch Subtraktion des gleitenden Durchschnitts der horizontalen Geschwindigkeit von der horizontalen Geschwindigkeit erhalten wird, als Beurteilungswert festsetzt.

6. Elektronische Vorrichtung (100) nach Anspruch 1, wobei die Steuerung (110) mindestens basierend auf der bestimmten Armschwungrichtung einen Bodenkontaktfuß des Benutzers bestimmt.

7. Elektronische Vorrichtung (100) nach Anspruch 6, wobei
die Steuerung (110) einen Zeitpunkt eines Bodenkontakts eines Fußes des Benutzers ermittelt und basierend auf dem ermittelten Zeitpunkt des Bodenkontakts und der bestimmten Armschwungrichtung den Bodenkontaktfuß des Benutzers bestimmt.

8. Elektronische Vorrichtung (100) nach Anspruch 7, wobei die Steuerung (110) bestimmt, dass ein Fuß auf einer selben Seite wie der tragende Arm Bodenkontakt herstellt, wenn in einem Zeitraum von dem ermittelten Zeitpunkt des Bodenkontakts bis zu einem Zeitpunkt eines nächsten Bodenkontakts eine Zeitspanne, in der der Beurteilungswert einen positiven Wert annimmt, größer ist als eine Zeitspanne, in der der Beurteilungswert einen negativen Wert annimmt.

9. Verfahren zur Bestimmung der Armschwungrichtung, bei dem eine informationsverarbeitende Vorrichtung an einem Arm eines Benutzers getragen wird und einen Detektor (160), der Beschleunigungskomponenten des Benutzers in jeder Richtung von drei zueinander orthogonalen Achsen ermittelt, und eine Steuerung (110) beinhaltet, wobei das Verfahren
**dadurch gekennzeichnet ist, dass** es Folgendes umfasst:
Bestimmen, durch die Steuerung (110) und basierend auf den durch den Detektor (160) ermittelten Beschleunigungskomponenten, einer Armschwungrichtung des Benutzers, wobei das Bestimmen Folgendes beinhaltet:

Erfassen, basierend auf den von dem Detektor (160) ermittelten Beschleunigungskomponenten auf den drei Achsen, einer horizontalen Geschwindigkeit, die eine horizontale Geschwindigkeitskomponente ist,
mit der erfassten horizontalen Geschwindigkeit als Beurteilungswert, Bestimmen, basierend auf dem Beurteilungswert, der Armschwungrichtung des Benutzers,
Aufteilen der Beschleunigungskomponenten auf den drei Achsen in eine erste Beschleunigungskomponente, die eine Beschleunigungskomponente auf einer ersten, aus den drei Achsen ausgewählten Achse ist, und eine zweite Beschleunigungskomponente, die eine Komponente einer Summe der Beschleunigungskomponenten von zwei anderen Achsen als der ersten Achse ist, und
Erfassen, basierend auf der ersten Beschleunigungskomponente und der zweiten Beschleunigungskomponente, der horizontalen Geschwindigkeit.

10. Computerlesbares Speichermedium, das ein Programm enthält,
**gekennzeichnet durch** Veranlassen einer Steuerung (110) einer informationsverarbeitenden Vorrichtung, die dazu konfiguriert ist, an einem Arm eines Benutzers getragen zu werden, und die einen Detektor (160), der dazu konfiguriert ist, Beschleunigungskomponenten des Benutzers in jeder Richtung von drei Achsen, die orthogonal zueinander sind, zu ermitteln, und die Steuerung (110) beinhaltet, eine Verarbeitung zu Folgendem auszuführen:

Bestimmen, basierend auf den von dem Detektor (160) ermittelten Beschleunigungskomponenten, einer Armschwungrichtung des Benutzers,
wobei das Bestimmen Folgendes beinhaltet:

Erfassen, basierend auf den von dem Detektor (160) ermittelten Beschleunigungskomponenten auf den drei Achsen, einer horizontalen Geschwindigkeit, die eine horizontale Geschwindigkeitskomponente ist,
mit der erfassten horizontalen Geschwindigkeit als Beurteilungswert, Bestimmen, basierend auf dem Beurteilungswert, der Armschwungrichtung des Benutzers,
Aufteilen der Beschleunigungskomponenten auf den drei Achsen in eine erste Beschleunigungskomponente, die eine Beschleunigungskomponente auf einer ersten, aus den drei Achsen ausgewählten Achse ist, und eine zweite Beschleunigungskomponente, die eine Komponente einer Summe der Beschleunigungskomponenten von zwei anderen Achsen als der ersten Achse ist, und

Erfassen, basierend auf der ersten Beschleunigungskomponente und der zweiten Beschleunigungskomponente, der horizontalen Geschwindigkeit.

## Revendications

**1.** Dispositif électronique (100) configuré pour être porté sur un bras d'un utilisateur, le dispositif électronique (100) comprenant :

un détecteur (160) configuré pour détecter des composantes d'accélération de l'utilisateur dans chaque direction de trois axes qui sont orthogonaux les uns aux autres et
un dispositif de commande (110),
**caractérisé en ce que** le dispositif de commande (110) est configuré pour déterminer, sur la base des composantes d'accélération détectées par le détecteur (160), une direction de balancement de bras de l'utilisateur, dans lequel le dispositif de commande (110) est configuré pour
acquérir, sur la base des composantes d'accélération sur les trois axes détectées par le détecteur (160), une vitesse horizontale qui est une composante de vitesse horizontale,
avec la vitesse horizontale acquise comme valeur de jugement, déterminer, sur la base de la valeur de jugement, la direction de balancement de bras de l'utilisateur,
diviser les composantes d'accélération sur les trois axes en une première composante d'accélération qui est une composante d'accélération sur un premier axe choisi parmi les trois axes, et une seconde composante d'accélération qui est une composante d'une somme des composantes d'accélération de deux axes autres que le premier axe, et
acquérir, sur la base de la première composante d'accélération et de la seconde composante d'accélération, la vitesse horizontale.

**2.** Dispositif électronique (100) selon la revendication 1, dans lequel le dispositif de commande (110)

calcule, sur la base d'un premier signal de nivellement obtenu en coupant des composantes haute-fréquence supérieures ou égales à une première fréquence de référence dans un premier signal qui est un signal exprimant des données chronologiques de la première composante d'accélération et d'un second signal de nivellement obtenu en coupant les composantes haute-fréquence supérieures ou égales à la première fréquence de référence dans un second signal qui est un signal exprimant des données chronologiques de la seconde composante d'accélération, une accélération horizontale, et
acquiert la vitesse horizontale à partir de l'accélération horizontale calculée.

**3.** Dispositif électronique (100) selon la revendication 2, dans lequel le dispositif de commande (110) acquiert la vitesse horizontale à partir d'un signal horizontal de nivellement obtenu en coupant des composantes haute-fréquence supérieures ou égales à une seconde fréquence de référence dans un signal exprimant des données chronologiques de l'accélération horizontale.

**4.** Dispositif électronique (100) selon la revendication 3, dans lequel le dispositif de commande (110)

calcule une moyenne mobile d'accélération horizontale qui est une moyenne mobile du signal horizontal de nivellement, et
acquiert la vitesse horizontale en intégrant une valeur obtenue en soustrayant la moyenne mobile d'accélération horizontale du signal horizontal de nivellement.

**5.** Dispositif électronique (100) selon la revendication 4, dans lequel le dispositif de commande (110)

calcule une moyenne mobile de vitesse horizontale qui est une moyenne mobile de la vitesse horizontale, et
établit une valeur obtenue en soustrayant la moyenne mobile de vitesse horizontale de la vitesse horizontale en tant que valeur de jugement.

**6.** Dispositif électronique (100) selon la revendication 1, dans lequel le dispositif de commande (110) détermine, sur la base au moins de la direction de balancement de bras déterminée, un pied en contact avec le sol de l'utilisateur.

**7.** Dispositif électronique (100) selon la revendication 6, dans lequel

le dispositif de commande (110) détecte un moment d'un contact avec le sol d'un pied de l'utilisateur et détermine, sur la base du moment détecté du contact avec le sol et de la direction de balancement de bras déterminée, le pied en contact avec le sol de l'utilisateur.

**8.** Dispositif électronique (100) selon la revendication 7, dans lequel le dispositif de commande (110) détermine qu'un pied sur un même côté que le bras porteur entre en contact avec le sol lorsque, au cours d'une période allant du moment détecté du contact avec le sol à un moment d'un contact suivant avec le sol, un laps de temps pendant lequel la valeur de jugement prend une valeur positive est supérieur à un laps de temps pendant lequel la valeur du jugement prend une valeur négative.

**9.** Procédé de détermination de direction de balancement de bras dans lequel un dispositif de traitement d'informations est porté sur un bras d'un utilisateur et comporte un détecteur (160) qui détecte des composantes d'accélération de l'utilisateur dans chaque direction de trois axes orthogonaux les uns aux autres, et un dispositif de commande (110), le procédé étant **caractérisé en ce qu'**il comprend :

la détermination, par le dispositif de commande (110) et sur la base des composantes d'accélération détectées par le détecteur (160), d'une direction de balancement de bras de l'utilisateur, dans lequel la détermination comporte l'acquisition, sur la base des composantes d'accélération sur les trois axes détectées par le détecteur (160), d'une vitesse horizontale qui est une composante de vitesse horizontale, avec la vitesse horizontale acquise comme valeur de jugement, la détermination, sur la base de la valeur de jugement, de la direction de balancement de bras de l'utilisateur, la division des composantes d'accélération sur les trois axes en une première composante d'accélération qui est une composante d'accélération sur un premier axe choisi parmi les trois axes, et une seconde composante d'accélération qui est une composante d'une somme des composantes d'accélération de deux axes autres que le premier axe, et l'acquisition, sur la base de la première composante d'accélération et de la seconde composante d'accélération, de la vitesse horizontale.

**10.** Support de stockage lisible par ordinateur contenant un programme **caractérisé en ce qu'**il amène un dispositif de commande (110), d'un dispositif de traitement d'informations qui est configuré pour être porté sur un bras d'un utilisateur et qui comporte un détecteur (160) configuré pour détecter des composantes d'accélération de l'utilisateur dans chaque direction de trois axes qui sont orthogonaux les uns aux autres et le dispositif de commande (110), à exécuter un traitement destiné à :

déterminer, sur la base des composantes d'accélération détectées par le détecteur (160), une direction de balancement de bras de l'utilisateur, dans lequel la détermination comporte l'acquisition, sur la base des composantes d'accélération sur les trois axes détectées par le détecteur (160), d'une vitesse horizontale qui est une composante de vitesse horizontale, avec la vitesse horizontale acquise comme valeur de jugement, la détermination, sur la base de la valeur de jugement, de la direction de balancement de bras de l'utilisateur, la division des composantes d'accélération sur les trois axes en une première composante d'accélération qui est une composante d'accélération sur un premier axe choisi parmi les trois axes, et une seconde composante d'accélération qui est une composante d'une somme des composantes d'accélération de deux axes autres que le premier axe, et l'acquisition, sur la base de la première composante d'accélération et de la seconde composante d'accélération, de la vitesse horizontale.

# FIG. 1

100

ELECTRONIC DEVICE

CONTROLLER ~110

STORAGE ~120

INPUTTER ~130

OUTPUTTER ~140

COMMUNICATOR ~150

DETECTOR ~160

# FIG. 2

# FIG. 3

# FIG. 4

```
         ┌──────────────────────────┐
         │ ARM SWING DETERMINATION  │
         │       PROCESSING         │
         └──────────────────────────┘
```

S101

ACQUIRE ACCELERATION ON EACH OF x, y, AND z AXES

S102

CALCULATE x2 AXIS ACCELERATIONON xy PLANE FROM ACCELERATION ON EACH OF x AND y AXES

S103

APPLY LOW PASS FILTER TO CHRONOLOGICAL SIGNALS OF z AXIS AND x2 AXIS ACCELERATION, AND ACQUIRE FIRST AND SECOND LEVELING SIGNALS

S104

CALCULATE ANGLE v_ang FROM x2 AXIS OF COMBINED VECTOR Lv OF FIRST AND SECOND LEVELING SIGNALS

S105

CALCULATE ANGLE n_ang FROM NORM n OF COMBINED VECTOR vn OF ACCELERATION OF EACH OF x2 AXIS AND z AXIS

S106

CALCULATE HORIZONTAL ACCELERATION ha

S107

APPLY LOW PASS FILTER TO CHRONOLOGICAL SIGNAL OF HORIZONTAL ACCELERATION ha AND ACQUIRE LEVELING HORIZONTAL SIGNAL Lha

S108

CALCULATE HORIZONTAL VELOCITY hv FROM LEVELING HORIZONTAL SIGNAL Lha

S109

CALCULATE DETERMINATION VALUE JV FROM HORIZONTAL VELOCITY hv

S110

DETERMINE ARM SWING DIRECTION ON BASIS OF DETERMINATION VALUE JV

END

14

# FIG. 5

# FIG. 6

# FIG. 7

# FIG. 8

# FIG. 9

ACCELERATION

# FIG. 10

ACCELERATION

# FIG. 11

**EP 4 327 739 B1**

**Patent documents cited in the description**

- US 10371516 B2

- JP 2011090548 A